# EUROPEAN PATENT SPECIFICATION

(11) **EP 2 900 088 B1**
(45) Date of publication and mention of the grant of the patent: **30.11.2016**
(21) Application number: 13766985.9
(22) Date of filing: 27.09.2013
(51) Int. Cl.: A23L 2/52

(54) **LIQUID CONCENTRATED VITAMIN E COMPOSITIONS**
FLÜSSIGE KONZENTRIERTE VITAMIN-E-ZUSAMMENSETZUNGEN
COMPOSITIONS LIQUIDES DE VITAMINE E CONCENTRÉE

(30) Priority: 27.09.2012 EP 12186371
(43) Date of publication of application: 05.08.2015
(73) Proprietor: DSM IP Assets B.V., 6411 TE Heerlen (NL)
(72) Inventor: HUG, Denis, CH-4002 Basel (CH); BADOLATO BOENISCH, Gabriela, CH-4002 Basel (CH); VOELKER, Karl Manfred, CH-4002 Basel (CH)
(74) Representative: Kurt, Manfred
(86) International application number: PCT/EP2013/070175
(87) International publication number: WO 2014/049112

(56) References cited:
- WO-A1-01/70044
- WO-A1-98/15195
- WO-A1-03/063617
- WO-A1-2004/095952
- US-B1- 6 329 423
- DATABASE WPI Week 200243 Thomson Scientific, London, GB; AN 2002-398246 XP002705582, & JP 2002 020268 A (TAKEDA CHEM IND LTD) 23 January 2002 (2002-01-23)
- DATABASE CAPLUS [Online] CHEMICAL ABSTRACTS SERVICE, COLUMBUS, OHIO, US; 15 April 2008 (2008-04-15), XP002705583, Database accession no. 2008:459350 & CN 101 156 648 A (ZHONGSHAN TIANTIAN ANIMAL HEAL [CN]) 9 April 2008 (2008-04-09)

## Description

The present patent application relates to liquid concentrated dl-α-tocopherol acetate compositions. The liquid composition comprises dl-α-tocopherol acetate and at least 40 weight-% (wt-%) of at least one polyoxyethylene sorbitan monofatty acid ester and said liquid composition has an IU value of at least 0.30 IU/mg (based on the total weight of the liquid composition). Furthermore the present invention is related to the use of such compositions in liquid formulations (especially beverages, such as soft drinks), which are transparent (even after pasteurization).

The goal of the present invention was to find a way which allows to providing (highly) concentrated dl-α-tocopherol acetate compositions, which are easy to produce and which are stable as such and as well in further applications.

Concentrated vitamin E (and/or vitamin E derivatives) compositions are widely used for the production of any kind of end-market products, which are enriched with such vitamins. Such compositions should contain a high amount of the vitamin E (and/or vitamin E derivatives) so that they can be diluted according to the needs.

WO98/15195 describes and discloses an optically clear composition of fat soluble vitamins useful to fortify beverages as well as beverages, which have been nutritionally supplemented using the optically clear composition of fat soluble vitamins.

Furthermore such compositions must be storage stable under usual conditions. Furthermore when the compositions are incorporated into products then such products must be stable as well.

Furthermore it is advantageous that the composition does not comprise many ingredients, so that it can be incorporated into further products very easily.

Furthermore, it is advantageous when compositions allow to producing liquid products (i.e. beverages, like soft drinks) which are transparent. By the term transparent, we mean clear, non-turbid and non-opaque. In the description of the present invention we are giving a method of determining this property.

Surprisingly, we found that a liquid composition comprising
(i) dl-α-tocopherol acetate, and
(ii) at least 40 wt-%, based on the total weight of the liquid composition, of at least one polyoxyethylene sorbitan monofatty acid ester,
and wherein said liquid composition having an IU of at least 0.30/mg (based on the total weight of the liquid composition),
fullfills all the requirements mentioned above.

Therefore the present invention relates to a liquid composition (LC1) comprising
(i) dl-α-tocopherol acetate, and
(ii) at least 40 wt-%, based on the total weight of the liquid composition, of at least one polyoxyethylene sorbitan monofatty acid ester,
and wherein said liquid composition having an IU of at least 0.30/mg (based on the total weight of the liquid composition.

The International Unit (IU) is a unit of measurement for the amount of a substance, based on biological activity or effect. It is abbreviated as IU, as UI (Spanish unidad internacional or French unite internationale or Italian unità internazionale), or as IE (German Internationale Einheit, Danish International Enhed, Swedish Internationell Enhet). It is used to quantify vitamins and similar biologically active substances.

For vitamin E (and vitamin E derivatives), 1 IU is the biological equivalent of about 0.667 mg d-α-tocopherol (2/3 mg exactly), or of 1 mg of dl- α-tocopherol acetate. The method determining the very commonly used IU values is i.e. described in Leth et al, J.Nutr., 1977 107(12), 2236 - 2243, this reference is hereby incorporated by reference.

The IU units are as stated above related to the weight:
40 weight-% dl- α-tocopherol acetate equals 29 weight-% d- α- tocopherol acetate which then equals 0.40 IU/mg.
60 weight-% dl- α- tocopherol acetate equals 44 weight-% d- α- tocopherol acetate equals 0.60 IU/mg.

Preferred is a liquid composition (LC2) comprising
(i) dl-α-tocopherol acetate, and
(ii) at least 40 wt-%, based on the total weight of the liquid composition, of at least one polyoxyethylene sorbitan monofatty acid ester,
and wherein said liquid composition having an IU of at least 0.35/mg (based on the total weight of the liquid composition.

The liquid compositions of the present invention are microemulsion-type systems. This means that the inner phase has usually an average particle size (d50) of < 100 nm.

The present invention relates to a liquid composition comprising dl-α-tocopherol acetate.

Vitamin E is a generic term for eight fat-soluble compounds found in nature, which are divided into two groups; four are tocopherols and four are tocotrienols. They are identified by prefixes alpha-, beta-, gamma-, and delta-. Natural tocopherols occur in the RRR-configuration only. The synthetic form contains eight different stereoisomers and is called all-rac-α-tocopherol.

Vitamin E is available as dl-α-tocopherol acetate by chemical synthesis or as RRR-tocopherol acetate isolated as byproduct in oil deodorization followed by chemical transformations

Dl-α-tocopherol is prepared synthetically by condensation of tri-methylhydroquinone and isophytol

Vitamin E has powerful antioxidant activity; vitamin E prevents the propagation of lipid peroxidation, and protects proteins and DNA from oxidative stress; vitamin E's potential to prevent oxidation of lipoproteins (HDL, LDL) suggests a protective role in cardiovascular function; and epidemiological studies found positive associations between vitamin E intake and reduced risk for cardiovascular disease

All the liquid compositions according to the present invention always comprise at least one polyoxyethylene sorbitan monofatty acid ester.

The polyoxyethylene sorbitan monofatty acid ester is chosen from the group consisting of polyoxyethylene(20) sorbitan monolaurate, polyoxyethylene(20) sorbitan-monopalmitate, polyoxyethylene(20) sorbitan monostearate and polyoxyethylene(20) sorbitan monooleate.

These polyoxyethylene sorbitan monofatty acid esters are also known as for example Polysorbate 20 (Tween 20^{®}, Radiamuls^{®} 2137), Polysorbate 40 (Tween 40^{®}), Polysorbate 60 (Tween 60^{®}, Radiamuls^{®} 2147) and Polysorbate 80 (Tween 80^{®}, Lamesorb SMO 20^{®}, Radiamuls^{®} 2157). These compounds are available commercially from various producers.

More preferred are polysorbate 20 and polysorbate 80.

Most preferred is polysorbate 80.

Therefore the present invention preferably relates to a liquid composition (LC3) comprising
(i) dl-α-tocopherol acetate, and
(ii) at least 40 wt-%, based on the total weight of the liquid composition, of polysorbate 20 and/or polysorbate 80,
and wherein said liquid composition having an IU of at least 0.30/mg (based on the total weight of the liquid composition.

Even more preferred are compositions LC3', which are compositions LC3 having an IU of at least 0.35/mg (based on the total weight of the liquid composition).

More preferably the present invention relates to a liquid composition (LC4) comprising
(i) dl-α-tocopherol acetate, and
(ii) at least 40 wt-%, based on the total weight of the liquid composition, of polysorbate 80,
and wherein said liquid composition having an IU of at least 0.30/mg (based on the total weight of the liquid composition).

Even more preferred are compositions LC4', which are compositions LC4 having an IU of at least 0.35/mg (based on the total weight of the liquid composition.

It is possible that the liquid composition according to present invention (LC1, LC2, LC3, LC3', LC4 and LC4') also comprises other ingredients than (i) and (ii). These non-essential ingredients can be any usually used ones, such as dyes, fragrances, fillers, buffers etc.

But the preferred embodiments of the present invention do not (substantially) comprise any further ingredients than those listed under (i) and (ii)

The liquid formulation according to the present invention are produced using commonly know processes.

The liquid compositions are prepared according to well known processes.

Usually they are produced as following:
The polysorbate (or a mixture of polysorbates) is filled in a temperature controlled vessel and the temperature is adjusted to 60°C.
DI-α-tocopherol acetate is heated up to 60°C and then it is added to the polysorbate (or a mixture of polysorbates) within 5 min under stirring. The stirrer speed is kept slow (100-300 rpm).

This solution is then slowly stirred for additionally 15 min at 60°C.
A clear slightly yellowish solution is obtained. The solution is free flowing at room temperature (RT).

As mentioned above the liquid compositions (LC1, LC2, LC3, LC3', LC4 and LC4') are used in the production of food products, feed products, dietary supplements and/or pharmaceutical products. These products are preferably in a liquid form.

The above disclosed and described LC1, LC2, LC3, LC3', LC4 and LC4" are preferably used in liquid formulations, such as beverages (especially clear beverages).

These liquid formulations, such as beverages (especially clear beverages) have a further advantage of being transparent. This means the liquid composition does not blur a liquid formulation, so that it can be used in many liquid products.

The liquid compositions LC1, LC2, LC3, LC3', LC4 and LC4' can be added to the products as in amount that the desired amount of dl-α-tocopherol acetate is obtained.

But it also possible (usually preferred) that the liquid compositions are diluted (to a so called stock solution) and then this diluted form is used for adjusting the desired concentration in the products.

For the liquid composition according to the present invention it is preferred to produce stock solution (diluted with water). Furthermore, it is preferred that the dilution process is carried out at elevated temperature (up to 50°C).

The measurement of the turbidity is done by using standard methods (EN27027(ISO7027). All the measurements for this patent application are done by using a turbiditymeter Hach 2100N IS^{®} from Hach Company, Loveland, Colorado (USA). The turbidity is given in nephelometric turbidity units (NTU). The measurement angle was 90° +-2,5° and the measurement wavelength was: 860nm +-10nm LED. The measurements were done at room temperature.

The turbidity of the liquid, transparent and non-pasteurized formulations and of the liquid, transparent pasteurized formulations comprising at least one of the above described compositions is less than 20 NTU, preferably less than 10 NTU (for a 0.027 IU/g).

The invention also relates to food products, feed products, dietary supplements and/or pharmaceutical products comprising a liquid composition LC1, LC2, LC3, LC3', LC4 and LC4" as described above.

Preferably such a product is a liquid formulation preferably a beverage.

### Figures:

Fig.1. shows the appearance attributes of non-pasteurised, flavored water after 3 months of storage.
Fig.2. shows the appearance attributes of pasteurised, flavored water after 3 months of storage.
Fig.3. shows the appearance attributes of non-pasteurised, mineral drink after 3 months of storage.
Fig.4. shows the appearance attributes of pasteurised, mineral drink after 3 months of storage.

The following examples serve to illustrate the invention. If not otherwise stated all parts are given related to the weight and the temperature is given in degree Celsius.

### Examples

### Example 1

100 g of dl-α-tocopherol-acetate (from DSM Nutritional Products Ltd) were mixed with 100 g of polysorbate 80 (Lamesorb SMO 20 from Cognis).

A composition was obtained with a median particle size (d 0.5) of the inner phase of 52nm (measured by a laser light scattering). This liquid composition contains 0.50 IU/mg.

In Table 1 the amounts of the ingredients are listed.

**Table 1: Liquid composition of Example 1:**

| **Ingredients** | **wt**-% |
|---|---|
| dl-α-tocopherol-acetate | 50 |
| polysorbate 80 | 50 |

### Example 2

80 g of dl-α-tocopherol-acetate (from DSM Nutritional Products Ltd) were mixed with 120 g of polysorbate 80 (Lamesorb SMO 20 from Cognis).

A composition was obtained with a median particle size (d 0.5) of the inner phase of 56nm (measured by a laser light scattering). This liquid composition contains 0.40 IU/mg.

In Table 2 the amounts of the ingredients are listed.

**Table 2: Liquid composition of Example 2:**

| **Ingredients** | **wt**-% |
|---|---|
| dl-α-tocopherol-acetate | 40 |
| polysorbate 80 | 60 |

### Application Examples

The following examples show how the liquid compositions according to the present invention are used to produce liquid products.

For all of the following examples the turbidity was measured as follows:
Turbidity measurements and visual evaluations of the beverages were conducted after beverage preparation and after 14, 30, 60 and 90 days storage.
Turbidity measurements were conducted using a turbidimeter (Hach 2100N IS^{®}, USA) and turbidity values were given in NTU (nephelometric turbidity units-Neophelometer measures the light scattered by a sample in 90° from the incident light path).

### Example 4 - 5: flavoured water soft drinks

Table 4 shows the list of the ingredients for the formulations of examples 4 - 5. For these flavoured water soft drinks the same basic formulation has been produced. But the Vitamin E stock solutions were different (in example 4 the composition of example 1 was used, in example 5 the composition of example 2 was used. The IU value was the same for all these flavoured water soft drinks

**Table 4**

| **Ingredients** | **amount** |
|---|---|
| Sugar (fine crystalline) | 7.2 g |
| Aqueous citric acid (50% w/w)⁺ | 2.0 g |
| Potassium sorbate | 0.2 g |
| Ginger ale flavour | 0.1 g |
| Lemon flavour | 0.2g |
| Vitamin E stock solution ° | 2.68g |
| Water to | 1000 ml |

| | |
|---|---|
| ⁺ the aqueous citric acid was produced as follows: 100g citric acid were dissolved in 100g water by stirring (magnetic stirrer), the solution was stirred at a temperature of to 30°C to 40°C until the acid was completely dissolved. °The vitamin E stock solutions (comprising the liquid compositions of examples 1 - 2) were prepared as follows: (i) 2g of the liquid composition of Example 1 were mixed with 98g water at a temperature of 50°C. This stock solution had an IU value of 0.01 IU/g. (used in Example 4) (ii) 2.5g of the liquid composition of Example 2 were mixed with 97.5g water at a temperature of 50°C. This stock solution had an IU value of 0.01 IU/g. (used in Example 5) | |

40 g water was put into a 1l flask, then 7.2 g sugar (fine crystalline) and 0.2 g potassium sorbate were added and the solution was stirred by using a magnetic stirrer.

Afterwards 0.1 g of Ginger ale flavour (Givaudan, 60131-76) and 0.2 g of lemon flavor (Givaudan, 78839-76) were added.

Then 2.68 g of the vitamin E stock solutions were added. Afterwards 900 g water were added followed by 2 g of the aqueous citric.

Finally the rest of the water was added so that 1000ml of the drink was obtained. This liquid formulation had a IU value of 0.027 IU/g.

The drink was filled in a transparent glass bottles (0,3 I) which were then closed with a crown cap.

To test the storage stability of the liquid formulation, the bottled formulations have been stored as such as well some of these liquid formulations which have been pasteurised.

The pasteurization process was carried out as follows:
The bottles containing the flavoured waters of Examples 4 - 5 were pasteurized for approximately 1 min at 80 °C using a tunnel pasteurizer (Miele, Switzerland).

The bottles (non-pasteurised and pasteurised) have been stored at a temperature of 25°C for 90 days. The NTU values have been measured (using the apparatus as stated above) after the preparation, after 14 days, 30 days, 60 days and 90 days.

The measured NTU values of the non-pasteurised examples 4 - 5 are listed in the following table 5:

**Table 5: Turbidity measurements of non pasteurized flavoured water samples**

| **Exp.** | **t=0** | **t= 14 days** | **t= 30 days** | **t= 60 days** | **t= 90 days** |
|---|---|---|---|---|---|
| **4** | 2.0 | 1.3 | 1.4 | 1.5 | 1.6 |
| **5** | 1.0 | 0.7 | 0.7 | 0.7 | 0.8 |

The measured NTU values of the pasteurised examples 4 - 5 are listed in the following table 6:

**Table 6: Turbidity measurements of non pasteurized flavoured water samples**

| **Exp.** | **t=0** | **t= 14 days** | **t= 30 days** | **t= 60 days** | **t= 90 days** |
|---|---|---|---|---|---|
| **4** | 1.3 | 1.2 | 1.2 | 1.3 | 1.5 |
| **5** | 0.7 | 0.7 | 0.6 | 0.7 | 0.9 |

### Example 7 - 8: mineral drinks

Table 7 shows the list of the ingredients for the formulations of examples 4 - 5. For these mineral drinks the same basic formulation has been produced. But the Vitamin E stock solutions were different (in example 7 the composition of example 1 was used, in example 8 the composition of example 2 was used. The IU value was the same for all these mineral drinks.

**Table 7**

| **Ingredients** | **amount** |
|---|---|
| Sugar syrup^{*} | 156.2 g |
| Aqueous citric acid (50% w/w)⁺ | 5.0 g |
| Potassium sorbate | 0.2 g |
| Ascorbic acid fine powder | 0.2 g |
| Apricot flavour | 0.2 g |
| Sodium chloride | 0.8 g |
| Calcium phosphate | 0.8 g |
| Magnesium citrate | 0.6 g |
| Potassium phosphate | 0.42 g |
| Vitamin E stock solution ° | 2.68g |
| Water to | 1000 ml |

| | |
|---|---|
| *640g sugar was added to 360g water in a stainless steel pot. The weight of pot, sugar and water was taken. The mixture was boiled gently, then let cool to ambient temperature. Afterward the evaporated water was replaced. *,° both solution were prepare in analogy to those in Examples 4 and 5. | |

The sugar syrup was put in a 1l volumetric flask then 0.2 g of ascorbic acid, 5.0 g of citric acid solution (50% w/w), 0.2 g of apricot flavour (Givaudan, 78848-56), 0.8 g of sodium chloride, 0.8 g of calcium phosphate, 0.6 g of magnesium citrate and 0.2 g of potassium phosphate were added. After each addition, the beverage was agitated by using a magnetic stirrer.

Afterward the vitamin E stock solution was added. Then the water was added to fill up to 1000ml.

The drink was filled in a transparent glass bottles (0,3 l) which were then closed with a crown cap.

To test the storage stability of the liquid formulation, the bottled formulations have been stored as such as well some of these liquid formulations which have been pasteurised.

The pasteurization process was carried in analogy to examples 4 - 5:
The bottles (non-pasteurised and pasteurised) have been stored at a temperature of 25°C for 90 days. The NTU values have been measured (using the apparatus as stated above) after the preparation, after 14 days, 30 days, 60 days and 90 days.

The measured NTU values of the non-pasteurised examples 7 - 8 are listed in the following table 8:

**Table 8: Turbidity measurements of non pasteurized mineral drink samples**

| **Exp.** | **t=0** | **t= 14 days** | **t= 30 days** | **t= 60 days** | **t= 90 days** |
|---|---|---|---|---|---|
| **7** | 1.8 | 2.1 | 3.0 | 4.0 | 5.1 |
| **8** | 1.2 | 1.6 | 2.1 | 3.0 | 4.3 |

The measured NTU values of the pasteurised examples 7 - 8 are listed in the following table 9:

**Table 9: Turbidity measurements of non pasteurized flavoured water samples**

| **Exp.** | **t=0** | **t= 14 days** | **t= 30 days** | **t= 60 days** | **t= 90 days** |
|---|---|---|---|---|---|
| **7** | 3.4 | 3.9 | 4.3 | 5.2 | 5.7 |
| **8** | 2.0 | 2.3 | 2.7 | 3.6 | 4.3 |

### Further tests:

The visual appearance of the liquid formulations (flavoured water and mineral drink formulations) has also been determined.

The ring formation (at the top), Particles on surface, Film formation on surface and sediment (on the bottom or in ring form) have been determined using the following ranking (6 is always very good and 1 is very bad.

### Ring formation:

6 = no ring
5 = hardly noticeable ring
4 = recognisable ring
3 = clear fine ring recognisable
2 = strong ring recognisable
1 = broad ring recognisable

### Particles on surface:

6 = no particles
5 = 1 to 10 particles
4 = > 10 particles
3 = not countable
2 = half of the surface covered with particles
1 = more than the half of the surface covered with particles

### Film forming on surface:

6 = no film visible
5 = hardly noticeable film
4 = clearly visible film
3 = slightly covering film
2 = covering film
1 = completely covering

### Sediment (on the bottom or in ring form):

6 = no sediment
5 = slight matt glimmer or hardly noticeable ring
4 = fine matt sediment or thin ring
3 = matt sediment or obvious fine ring visible
2 = strong matt sediment or strong ring visible
1 = very strong matt sediment or broad ring visible

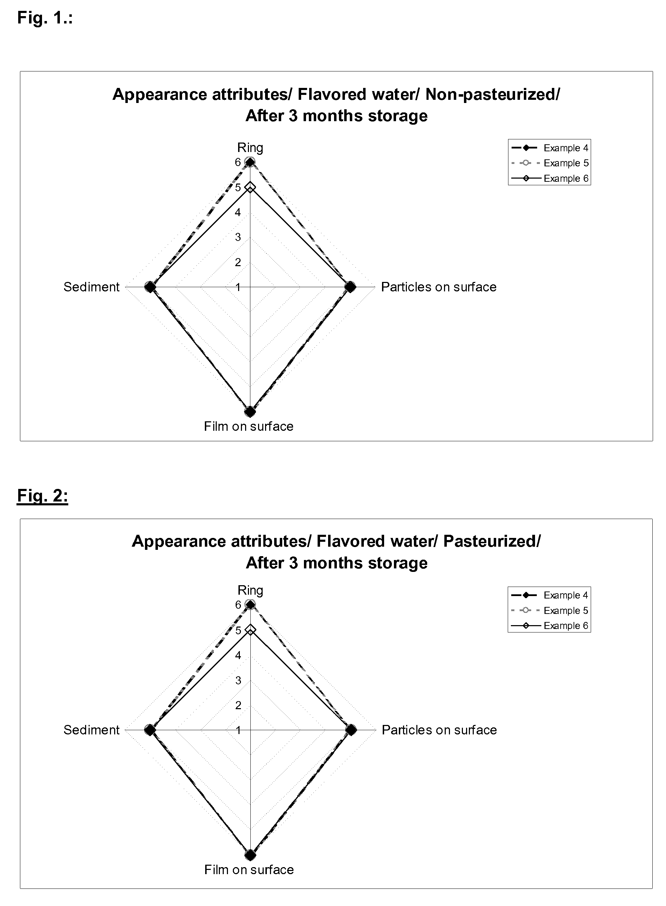

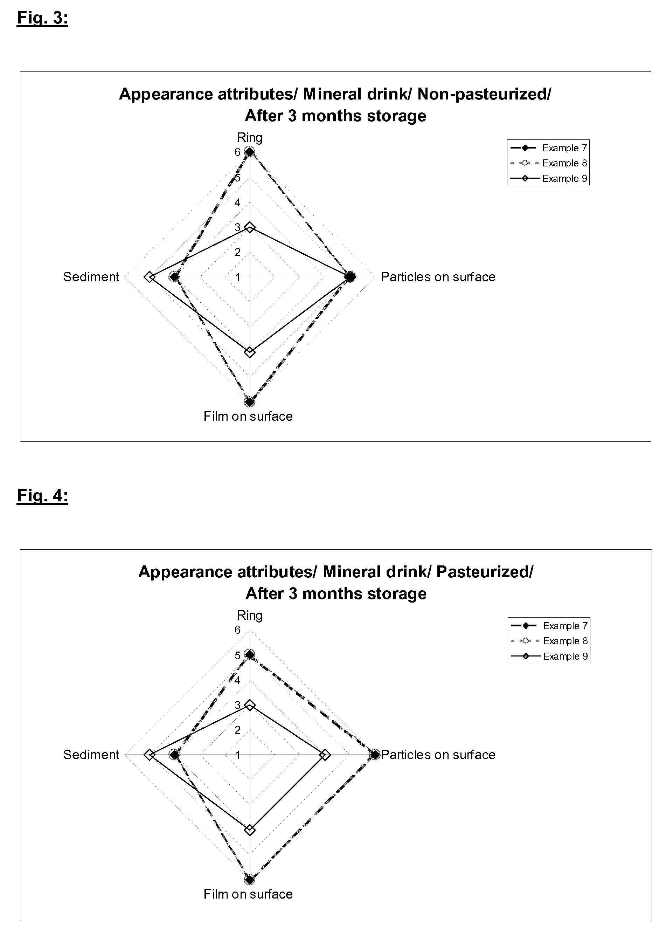

The results, which are good (excellent for the polysorbate 80 compositions) are visualised in Figures 1 - 4 of this patent application.

### Ring formation:

6 = no ring
5 = hardly noticeable ring
4 = recognisable ring
3 = clear fine ring recognisable
2 = strong ring recognisable
1 = broad ring recognisable

### Particles on surface:

6 = no particles
5 = 1 to 10 particles
4 = > 10 particles
3 = not countable
2 = half of the surface covered with particles
1 = more than the half of the surface covered with particles

### Film forming on surface:

6 = no film visible
5 = hardly noticeable film
4 = clearly visible film
3 = slightly covering film
2 = covering film
1 = completely covering

### Sediment (on the bottom or in ring form):

6 = no sediment
5 = slight matt glimmer or hardly noticeable ring
4 = fine matt sediment or thin ring
3 = matt sediment or obvious fine ring visible
2 = strong matt sediment or strong ring visible
1 = very strong matt sediment or broad ring visible

The results, which are good (excellent for the polysorbate 80 compositions) are visualised in Figures 1 - 4 of this patent application.

## Claims

1. A liquid composition comprising
(i) dl-α-tocopherol acetate, and
(ii) at least 40 wt-%, based on the total weight of the liquid composition, of at least one polyoxyethylene sorbitan monofatty acid ester,
and wherein said liquid composition having an IU of at least 0.30/mg (based on the total weight of the liquid composition).

2. The liquid composition according to claim 1, wherein said liquid composition having an IU of at least 0.35/mg (based on the total weight of the liquid composition).

3. The liquid composition according to anyone of the preceding claims, wherein the polyoxyethylene sorbitan monofatty acid ester is chosen from the group consisting of polyoxyethylene(20) sorbitan monolaurate, polyoxyethylene(20) sorbitan-monopalmitate, polyoxyethylene(20) sorbitan monostearate and polyoxyethylene(20) sorbitan monooleate.

4. The liquid composition according to anyone of the preceding claims, wherein the polyoxyethylene sorbitan monofatty acid ester is chosen from the group consisting of polysorbate 20 and polysorbate 80.

5. The liquid composition according to anyone of the preceding claims, wherein the polyoxyethylene sorbitan monofatty acid ester is polysorbate 80.

6. Use of a liquid composition according to anyone of the preceding claims in the production of food products, feed products, dietary supplements and/or pharmaceutical products.

7. Use according to claim 6, wherein product is a liquid formulation preferably a beverage.

8. Food products, feed products, dietary supplements and/or pharmaceutical products comprising a liquid composition according to anyone to anyone of claims 1 - 5.

9. Product according to claim 8, which is a liquid formulation preferably a beverage.

## Patentansprüche

1. Flüssige Zusammensetzung, die
(i) dl-α-Tocopherylacetat und
(ii) mindestens 40 Gew.-% in Bezug auf das Gesamtgewicht der flüssigen Zusammensetzung an mindestens einem Polyoxyethylen-Sorbitan-Monofettsäureester umfasst,
und wobei die flüssige Zusammensetzung einen IU-Wert von mindestens 0,30/mg (in Bezug auf das Gesamtgewicht der flüssigen Zusammensetzung) aufweist.

2. Flüssige Zusammensetzung nach Anspruch 1, wobei die flüssige Zusammensetzung IU-Wert von mindestens 0,35/mg (in Bezug auf das Gesamtgewicht der flüssigen Zusammensetzung) aufweist.

3. Flüssige Zusammensetzung nach einem der vorhergehenden Ansprüche, wobei der Polyoxyethylen-Sorbitan-Monofettsäureester aus der Gruppe bestehend aus Polyoxyethylen(20)-Sorbitan-Monolaurat, Polyoxyethylen(20)-Sorbitan-Monopalmitat, Polyoxyethylen(20)-Sorbitan-Monostearat und Polyoxyethylen(20)-Sorbitan-Monooleat ausgewählt ist.

4. Flüssige Zusammensetzung nach einem der vorhergehenden Ansprüche, wobei der Polyoxyethylen-Sorbitan-Monofettsäureester aus der Gruppe bestehend aus Polysorbat 20 und Polysorbat 80 ausgewählt ist.

5. Flüssige Zusammensetzung nach einem der vorhergehenden Ansprüche, wobei es sich bei dem Polyoxyethylen-Sorbitan-Monofettsäureester um Polysorbat 80 handelt.

6. Verwendung einer flüssigen Zusammensetzung nach einem der vorhergehenden Ansprüche in der Herstellung von Nahrungsmittelprodukten, Futtermittelprodukten, Nahrungsmittelergänzungsstoffen und/oder pharmazeutischen Produkten.

7. Verwendung nach Anspruch 6, wobei es sich bei dem Produkt um eine flüssige Formulierung, vorzugsweise ein Getränk, handelt.

8. Nahrungsmittelprodukte, Futtermittelprodukte, Nahrungsmittelergänzungsstoffe und/oder pharmazeutische Produkte, die eine flüssige Zusammensetzung nach einem der Ansprüche 1-5 umfassen.

9. Produkt nach Anspruch 8, bei dem es sich um eine flüssige Formulierung, vorzugsweise ein Getränk, handelt.

## Revendications

1. Composition liquide, comprenant
(i) de l'acétate de dl-α-tocophérol, et
(ii) au moins 40% en poids, sur la base du poids total de la composition liquide, d'au moins un ester de monoacide gras et de sorbitane polyoxyéthyléné,
et où ladite composition liquide possède une UI d'au moins 0,30/mg (sur la base du poids total de la composition liquide).

2. Composition liquide selon la revendication 1, où ladite composition liquide possède une UI d'au moins 0,35/mg (sur la base du poids total de la composition liquide).

3. Composition liquide selon l'une quelconque des revendications précédentes, dans laquelle l'ester de monoacide gras et de sorbitane polyoxyéthyléné est choisi dans le groupe constitué par le monolaurate de sorbitane et de polyoxyéthylène (20), le monopalmitate de sorbitane et de polyoxyéthylène (20), le monostéarate de sorbitane et de polyoxyéthylène (20) et le monooléate de sorbitane et de polyoxyéthylène (20).

4. Composition liquide selon l'une quelconque des revendications précédentes, dans laquelle l'ester de monoacide gras et de sorbitane polyoxyéthyléné est choisi dans le groupe constitué par le polysorbate 20 et le polysorbate 80.

5. Composition liquide selon l'une quelconque des revendications précédentes, dans laquelle l'ester de monoacide gras et de sorbitane polyoxyéthyléné est le polysorbate 80.

6. Utilisation d'une composition liquide selon l'une quelconque des revendications précédentes, dans la production de produits alimentaires, de produits d'aliments pour animaux, de compléments diététiques et/ou de produits pharmaceutiques.

7. Utilisation selon la revendication 6, dans laquelle le produit est une formulation liquide, préférablement une boisson.

8. Produits alimentaires, produits d'aliments pour animaux, compléments diététiques et/ou produits pharmaceutiques, comprenant une composition liquide selon l'une quelconque des revendications 1-5.

9. Produit selon la revendication 8, qui est une formulation liquide, préférablement une boisson.
